# EUROPEAN PATENT APPLICATION

(11) **EP 4 238 475 A1**
(43) Date of publication of application: **06.09.2023**
(21) Application number: 23157977.2
(22) Date of filing: 22.02.2023
(51) Int. Cl.: A61B 1/00, A61B 1/12

(54) **ALBARRAN**

(30) Priority: 02.03.2022 US 202263315709 P
(71) Applicant: Olympus Winter & Ibe GmbH, 22045 Hamburg (DE)
(72) Inventor: Schmuck, Irina, 25746 Heide (DE); Schröder, Bastian, 22589 Hamburg (DE); Schulz, Kevin Alexander, 22889 Wilstedt (DE); Rühs, Andreas, 22926 Ahrensburg (DE)
(74) Representative: Hoener, Matthias

(57) **Abstract**

The invention is directed on an albarran (10) that can be cleaned or reprocessed particularly thoroughly. This is achieved by arranging at least one flushing port (31) on a drive body (18) of the albarran (10). A flushing line can be connected to this flushing port (31) during cleaning or reprocessing. The rinsing liquid can be introduced in a targeted manner through this defined access into the interior of the drive body (18) or into the gear chamber (24).

## Description

The invention relates to an albarran according to patent claim 1.

Albarrans are used as support for operations or treatments with surgical instruments, such as endoscopes, resectoscopes, cystoscopes or the like. An albarran can be used, for example, to deflect a flexible forceps within a patient in a targeted as well as controlled manner. For this purpose, the albarran, just like an endoscope for example, has a rod-like or tube-like shaft, which is to be guided into the patient's body with a distal end. At a proximal end of the albarran, outside the patient, the shaft is connected to a main body. Via this main body, further instruments or tools, such as optics, wires or the like, can be guided through the shaft into the patient via various openings or ports.

At the distal end of the shaft, the albarran has a lever, the so-called Albarran lever. This lever is designed to be movable and can be actuated or pivoted via a toggle on the main body. For this purpose, the lever is mechanically coupled to the toggle along the shaft. This mechanical coupling can be either a rod or a pull wire. Usually, the lever is connected to the toggle via two pull wires. It is conceivable that both pull wires serve equally to swing the lever back and forth or that different movements of the lever can be effected via the toggle using the two pull wires.

For reliable operation of the albarran lever, the pull wires are to be tensioned inside the main body and there in a drive body or in a gear chamber with a pull-wire carrier. The pull-wire carrier is mounted on the shaft so that it can be moved along a longitudinal axis of the albarran. The rotational movement of the toggle is converted in the gear chamber into a linear movement of the pull-wire carrier. This allows the toggle to be rotated to move the pull wire carrier, and thus the wires, back and forth parallel to the shaft, rotating the lever at the distal end of the shaft around an axis. The gear is located in the gear chamber or drive body and is sealed to the outside by grease, adhesive, or gaskets. This sealing means that the gear chamber has no access or contact with the patient or user.

For reprocessing of the albarran or the main body, it is essential that all components, especially internal components, can be rinsed, disinfected and sterilized. The sealing elements such as grease, adhesive and other seals suffer from the various reprocessing processes (ultrasonic bath, machine cleaning, sterilization in an autoclave). The grease can be washed out during these processes, adhesive can become brittle, and the seals can fatigue. The previously sealed gear chamber can thus become leaky as a result of the reprocessing processes. As a result, liquids can enter the gear chamber during the operation and contaminate it permanently. Removal of these contaminants is only possible with increased effort. It cannot be ruled out that some of the contamination will remain in the gear chamber during reprocessing of the instrument. As a result, there is a risk that when the albarran is used again, the residual liquid will escape from the gear chamber and cause a serious health hazard to the patient or the surgeon. Without being able to achieve the necessary thoroughness in reprocessing, surgical instruments such as the albarran described here and its components cannot be reused. However, offering or using such complex instruments as *single-use instruments* is too cost-intensive.

The invention is based on the problem of creating an Albarran that can be cleaned or reprocessed particularly thoroughly.

A solution to this problem is described by the features of claim 1. Accordingly, it is provided that at least one flushing port is arranged on a drive body of the albarran. A flushing line can be connected to this flushing port during cleaning or during reprocessing. Through this defined access into the interior of the drive unit or into the gear chamber, the flushing liquid, which may be provided with cleaning agent, can be introduced in a targeted manner. As a result, the gear chamber and the components arranged in the gear chamber, such as the pull- wire carrier, a gear unit and the wires, can be cleaned in a very thorough manner. The rinsing liquid can leave the gear chamber again through another opening in the drive unit.

A particularly preferred embodiment of the invention provides for the flushing port to enclose an acute angle with the shaft pointing in the distal direction of the albarran. As a result of this relative angular position, the flushing liquid flows into the gear chamber at an angle, namely in the proximal direction. It has been shown that this oblique inflow of the flushing liquid flushes the interior of the gear chamber as well as all components stored therein in a very efficient and thorough manner. In order to leave the gear or transmission chamber again, the flushing liquid must change its direction of flow within the gear chamber. The resulting turbulence and pressure differences within the flushing flow allow any contaminants and residues to be flushed out particularly thoroughly.

In particular, the invention provides for the flushing connection or port to be arranged directly above the pull-wire carrier on the drive body, so that the flushing fluid flow meets the pull-wire carrier, which is in particular wedge-shaped or trapezoidal, and is divided into two partial flows. This division of the flushing flow ensures that all surfaces within the gear chamber are reached. This prevents dead volumes within the drive body from being washed around.

Another advantageous embodiment of the present invention may provide that at least one inner wall of the gear chamber is convex in proximal direction. Preferably, this inner wall may be an inner wall of the gear chamber opposite the flushing connection. In the embodiment example shown and described here, the inner wall of the gear chamber would be convex in the proximal direction, or concave in the distal direction. Due to this design of the inner wall, the flushing fluid flowing through the flushing port into the gear chamber is reflected particularly efficiently at the inner wall, which is convexly curved in the proximal direction. The convex design of at least one wall of the gear chamber towards the outside allows particularly advantageous flushing of all components arranged inside the gear chamber. In addition, this curvature makes it possible to achieve local increases in the flow velocity, as a result of which particularly thorough cleaning of all surfaces can be achieved due to the resulting pressure difference.

Preferably, the main body has an outlet opening at its distal end, in particular parallel to the shaft. This outlet opening is aligned in the same distal direction as the flushing port. Thus, the flushing flow within the drive body must perform a change of direction of at least nearly 180°. This necessary reversal of direction of the flushing liquid has a particularly beneficial effect on the cleaning action of the flushing liquid.

Preferably, it can be provided that the outlet opening is larger or the same size as the flushing opening. These differently dimensioned openings create a pressure gradient which has an advantageous effect on the flow rate of the rinsing liquid.

Another advantageous embodiment of the invention may provide that the outlet opening is tubular and tapers in diameter in the distal direction. This tapering results in an increase in the flow velocity, which creates a kind of suction effect inside the drive body. As a result, even larger contaminants, such as particles, can be efficiently removed from the transmission chamber.

It is further conceivable according to the invention that at least one, preferably two, closable accesses are arranged on the main body. These accesses can be used to introduce further instruments or tools into the shaft during the operation. Similarly, these accesses can be used to serve as an outlet for the rinsing fluid. Similarly, it is conceivable that an additional rinsing fluid is introduced into the gear chamber through the accesses.

It is conceivable that the flushing port is designed as a Lürlock port. This allows corresponding lines or hoses or other fluid-carrying means to be connected to the flushing port in a simple and safe manner. During operation, it is conceivable to close the flushing port by means of a sealing cap, a lid, a valve, a flap or the like. This can prevent liquid from escaping from the gear chamber, nor can it prevent liquid or other objects from entering the gear chamber. During the operation, all openings are sealed with these means. No other sealing agents are necessary. These agents can be easily removed for cleaning or reprocessing.

A preferred embodiment of the present invention is explained in more detail below with reference to the drawing. In this show:
- Fig. 1: a side view of an albarran,
- Fig. 2: a view of the albarran as shown in Fig. 1,
- Fig. 3: an illustration of a proximal end of the albarran,
- Fig. 4: a sectional view through the albarran according to Fig. 1,
- Fig. 5: a detail magnification of the proximal portion of the albarran shown in Fig. 4, and
- Fig. 6: a view into a main body of the albarran.

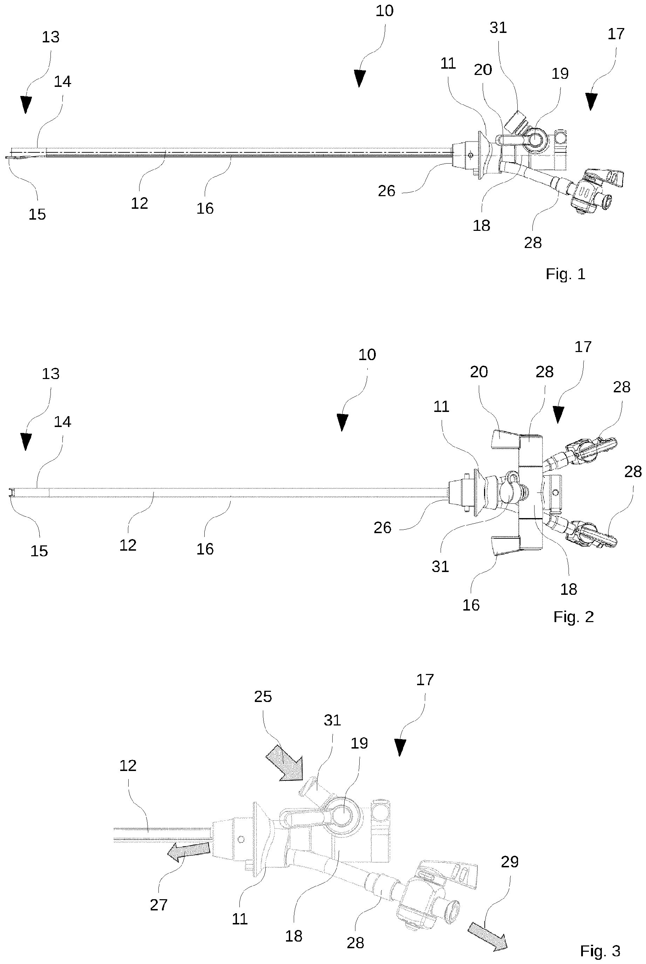

Fig. 1 shows a possible embodiment of an albarran 10. It should be expressly noted that the scope of protection of the present patent application is not intended to be limited to this embodiment. Rather, it is intended that further embodiments of the invention are also covered by the scope of protection.

The albarran 10 consists essentially of a main body 11 and a shaft 12 mounted on the main body 11. The shaft 12 is tubular and is guided into the body of the person with a distal end 13 in front for the treatment of persons. Various medical or surgical instruments can be passed through the shaft 12. An albarran lever 15 is movably arranged to the distal end 13 of the shaft 12 on an outer wall 14. This lever 15 serves the surgeon during treatment as an aid for carrying out further treatment steps. The lever 15 is mounted so that it can be pivoted around an axis. To move the lever 15 around its axis, it can be actuated via pull wires 16.

The pull wires 16 extend parallel to the shaft 12 from the lever 15 to the proximal end 17 of the albarran 10, passing through the main body 11 and being attached to a pull-wire carrier 21 in a drive body 18 of the main body 11. For actuating the lever 15, the drive body 18 has a toggle 19 with two actuating means 20. By rotating these actuating means 20, the pull-wire carrier 21 and thus also the pull wires 16 are moved linearly forwards or backwards along the shaft 12, thereby performing a pivoting movement of the lever 15. For the conversion of the rotational movement into a translational movement, the pull-wire carrier 21 has an elongated bore 22. A pin 23 of the toggle 19 engages this elongated bore 22, this pin 23 being eccentrically offset from the axis of rotation of the toggle 19. This combination of the bore 22 and the pin 23 is also referred to as the gear, for which reason the interior of the drive body 18 is also referred to as the gear chamber 24.

In order to be able to clean the albarran 10 and in particular the main body 11, a flushing port 31 is arranged on the drive body 18. This flushing port 31 forms an acute angle with the shaft 12 and is thus inclined in the direction of the distal end 13 of the shaft 12 (Figs. 1, 2). A flushing liquid can be conveyed through this flushing port 31 along the direction of arrow 25 into the drive body 18 (Fig. 3). The liquid can leave the main body 11 again, for example, at a distal end 26 in the direction of arrow 27, or can be discharged or sucked out via two additional ports 28 in the direction of arrow 29. While the flushing port 31 can be equipped with a lid for closing the opening, the accesses 28 can each have a valve in the embodiment example of the albarran 10 shown here. Via the access ports 28, the flushing liquid can be actively sucked out of the main body 11 by a pump. In this way, the main body 11 or the entire albarran 10 can be flushed in a particularly thorough manner.

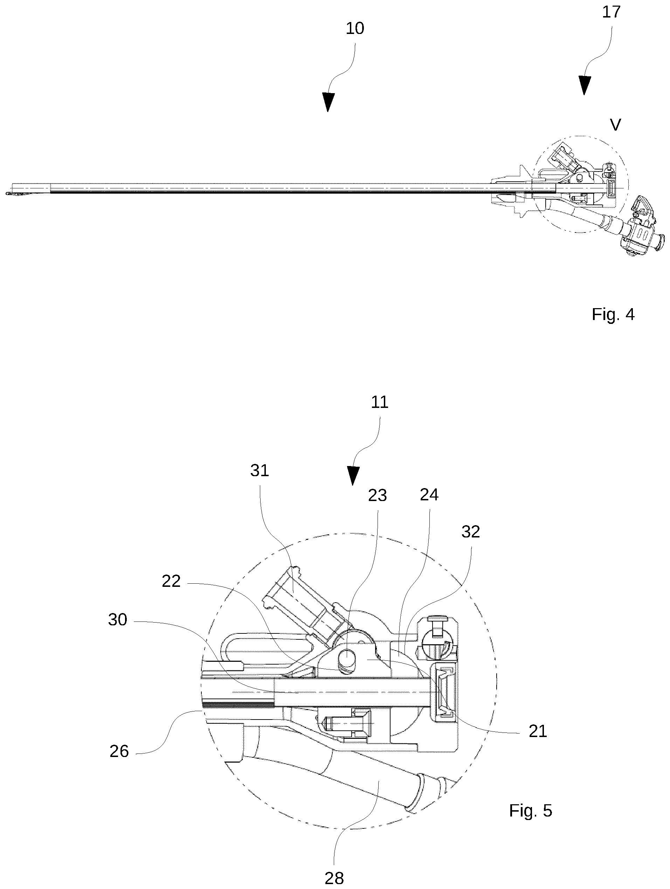

As the flushing fluid flows into the gear chamber 24, both the inner walls of the gear chamber 24 and the surfaces of an inner shaft 30 or optic tube and the pull-wire carrier 21 are cleaned. This cleaning is particularly efficient due to the dimensioning of the diameters of the flushing port 31 as well as the distal end 26 or the outlet and the accesses 28. An essential feature of the invention is that a proximal inner wall 32 of the gear chamber 24 is convex in the proximal direction (Figs. 4, 5). This convex curvature of the inner wall 32 deflects the flushing fluid flowing through the flushing port 31 into the gear chamber 24 in the opposite direction. This deflection rinses all surfaces within the gear chamber 24 in a very efficient manner. In addition, the rinsing fluid is returned directly in the direction of the distal end 26 of the main body 11.

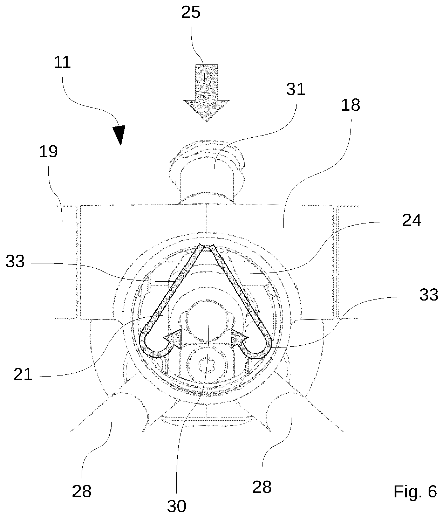

As shown in Fig. 6, the pull-wire carrier 21 may have a trapezoidal cross-section. This shape divides the flushing flow entering the gear chamber 24 through the flushing port 31 into two partial flows 33, so that the interior of the drive body 18 is flushed out evenly.

A hose can be coupled to the flushing port 31, which can be in the form of a Lürlock, for cleaning the albarran 10 and in particular the gear chamber 24. The flushing liquid can be conveyed into the gear chamber 24 via this hose under a certain pre-pressure. After completion of the cleaning process and sufficient drying of the albarran 10, the openings and in particular the flushing port 31 and the distal end 26 of the main body 11 are closed again so that no external substances can enter the gear chamber 24 during the subsequent operation or no external substances can enter the patient from the gear chamber 24.

### List of reference signs:

- 10: Albarran
- 11: Main body
- 12: Shaft
- 13: Distal end
- 14: Outer wall
- 15: Lever
- 16: Pull wire
- 17: Proximal end
- 18: Drive body
- 19: Toggle
- 20: Actuator
- 21: Pull-wire carrier
- 22: Bore
- 23: Pin
- 24: Gear chamber
- 25: Arrow direction
- 26: Distal end
- 27: Arrow direction
- 28: Access
- 29: Arrow direction
- 30: Inner shaft
- 31: Flushing port
- 32: Inner wall
- 33: Partial flow

## Claims

1. Albarran (10) with a tubular shaft (12), at the distal end (13) of which an Albarran lever (15) is arranged and at the proximal end (17) of which a main body (11) with a drive body (18) is arranged, wherein the Albarran lever (15) being movable by means of a toggle (19) on the drive body (18), and wherein the movement of the Albarran lever (15) being transferd from the toggle (19) by at least one, preferably two, pull wires (16) which are fastened within a gear chamber (24) of the drive body (18) to a pull-wire carrier (21), **characterized in that** a flushing port (31) is arranged on the drive body (18).

2. Albarran (10) of claim 1, **characterized in that** the flushing port (31) includes an acute angle with the shaft (12) facing in the distal direction of the albarran (10).

3. Albarran (10) according to claim 1 or 2, **characterized in that** the flushing port (31) is arranged directly above the pull-wire carrier (21) on the drive body (18), so that a flushing fluid stream impinges on the pull-wire carrier (21), which is in particular of wedge-shaped or trapezoidal design, and is divided into two partial streams.

4. Albarran (10) according to one of the preceding claims, **characterized in that** at least one inner wall (32) of the gear chamber (24) is convex in the proximal direction, in particular concave in the distal direction.

5. Albarran (10) according to one of the preceding claims, **characterized in that** an inner wall (32) of the gear chamber (24) opposite the flushing port (31) is convex, in particular concave in the distal direction.

6. Albarran (10) according to one of the preceding claims, **characterized in that** an inner wall (32) of the gear chamber (24) is convex in the proximal direction, in particular concave in the distal direction.

7. Albarran (10) according to any one of the preceding claims, **characterized in that** the main body (11) has an outlet opening at its distal end (26), preferably parallel to the shaft (12).

8. Albarran (10) according to claim 7, **characterized in that** the outlet opening is larger than or equal to the flushing port (31).

9. Albarran (10) according to claim 7 or 8, **characterized in that** the outlet opening is tubular and tapers in diameter in the distal direction.

10. Albarran (10) according to one of the preceding claims, **characterized in that** at least one, preferably two, closable accesses (28) are arranged on the main body (11).

11. Albarran (10) according to one of the preceding claims, **characterized in that** the flushing port (31) is designed as a Lürlock port.

12. Albarran (10) according to any one of the preceding claims, **characterized in that** the flushing port (31) is closable by a sealing cap, lid, valve, flap or the like.
